# FASCICULE DE BREVET EUROPEEN

(11) **EP 0 489 656 B1**
(45) Date de publication et mention de la délivrance du brevet: **06.09.1995**
(21) Numéro de dépôt: 91403282.6
(22) Date de dépôt: 04.12.1991
(51) Int. Cl.: G01N 33/24, E02D 1/02, E02D 1/06

(54) **Dispositif de mesure de la charge hydraulique et de la concentration ionique du sol**
Vorrichtung zur Messung des Porenwasserdrucks und der Ionenkonzentration des Bodens
Device for measuring the pore water pressure and ionic concentration of soil

(30) Priorité: 06.12.1990 FR 9015299
(43) Date de publication de la demande: 10.06.1992
(73) Titulaire: COMMISSARIAT A L'ENERGIE ATOMIQUE, 75015 Paris (FR)
(72) Inventeur: Moutonnet, Pierre, F-84120 Pertuis (FR); Guiraud, Gérard, F-13100 St Marc Jaumegarde (FR); Marol, Christine, F-13126 Vauvenargues (FR)
(74) Mandataire: Dubois-Chabert, Guy

(56) Documents cités:
- EP-A- 0 062 022
- AT-B- 390 289
- DE-A- 2 101 234
- DE-A- 3 911 151
- US-A- 4 068 525

## Description

### DISPOSITIF DE MESURE DE LA CHARGE HYDRAULIQUE ET DE LA CONCENTRATION IONIQUE DU SOL

La présente invention concerne un dispositif pour mesurer la charge hydraulique et la concentration ionique du sol. Elle trouve de nombreuses applications dans les domaines de l'hydrologie, de l'écologie, de l'agronomie, de l'hydrogéologie et de la physique du sol en général. De façon plus particulière, elle peut s'appliquer au dosage des engrais et à la détermination du degré de pollution du sol.

L'eau migre verticalement dans le sol sous l'action des gradients de charge hydraulique existant sur sa trajectoire. Dans sa course, elle entraîne différentes matières et sels dont certains ne sont pas réactifs avec le sol. On peut donc analyser les eaux du sol. Ce type d'analyse nécessite deux dispositifs :
- un tensiomètre pour mesurer la charge hydraulique du sol ; et
- un extracteur de solution pour mesurer la concentration ionique du sol.

Un tel dispositif d'extraction de solution existe sous la marque déposée "DTS 2 000" de la Société Nardeuxhumisol.

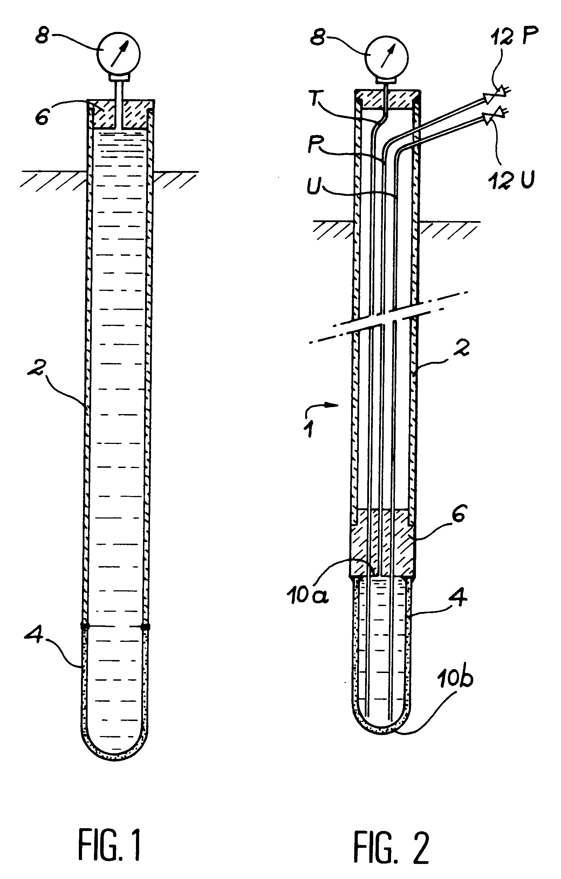

Sur la figure 1, on a représenté un tensiomètre selon l'art antérieur.

Ce tensiomètre comprend une canne guide 2 creuse à l'extrémité inférieure de laquelle est montée une bougie 4 en céramique poreuse.

Tout au long de la description, on comprendra par "bougie" une bougie filtrante, c'est-à-dire un cylindre creux et poreux.

Le rôle de la bougie 4 en céramique poreuse est de permettre la mise en équipotentiel hydraulique entre la solution du sol et l'intérieur de la canne guide. A l'extrémité supérieure de la canne guide, un bouchon 6 d'étanchéité assure la fermeture de la canne guide 2. Un manomètre 8 en liaison avec l'eau de la canne guide 2 est fixe sur ce bouchon 6 d'étanchéité.

Dans le cas de l'extracteur de solution, une pompe à vide, non représentée, est reliée à la canne guide 2 permettant ainsi de créer une forte dépression à l'intérieur de la canne guide 2 et de la bougie 4 en céramique poreuse permettant l'accumulation d'eau à l'intérieur de la bougie et de la canne guide.

Ces dispositifs présentent de nombreux inconvénients. En effet, le remplissage de la canne guide nécessite, non seulement un temps assez long, mais surtout, une importante quantité d'eau qui est drainée de façon irréversible.

Il y a également des risques de variation de la solution du sol : du fait de la forte dépression créée, l'eau du sol va migrer préférentiellement des zones à forte porosité, faisant ainsi varier la teneur en solutés de la solution du sol.

La présente invention permet de remédier à ces inconvénients.

Elle a justement pour objet un dispositif de mesure de la charge hydraulique et de la concentration ionique du sol utilisable de façon autonome sur le terrain.

De façon plus précise, l'invention a pour objet un dispositif de mesure de la charge hydraulique et de la concentration ionique du sol, comprenant une sonde comportant une canne guide, une bougie en céramique poreuse montée à l'extrémité inférieure de la canne guide, un bouchon d'étanchéité et un dispositif manomètrique, et des moyens, associés à ladite sonde, pour mesurer la charge hydraulique du sol, ainsi que la concentration des solutés, caractérisé en ce que la sonde comporte des tubes capillaires plongeant à l'intérieur de ladite sonde jusqu'à la bougie en céramique poreuse.

De façon avantageuse, les tubes capillaires sont au moins au nombre de trois, un premier tube capillaire servant à mesurer la charge hydraulique, un second tube capillaire assurant l'extraction du soluté à étudier et un troisième tube capillaire servant à purger de son air un circuit hydraulique comprenant lesdits tubes capillaires et la bougie en céramique poreuse.

Selon l'invention, le premier tube capillaire, relié par une extrémité supérieure au dispositif manométrique, pénètre jusque dans la partie inférieure de la bougie en céramique poreuse, le second tube capillaire, relié par une extrémité supérieure à une vanne assurant la fermeture ou l'ouverture dudit second tube capillaire, pénètre jusque dans la partie inférieure de la bougie en céramique poreuse, le troisième tube capillaire, relié par une extrémité supérieure à une vanne assurant la fermeture ou l'ouverture dudit troisième tube capillaire, pénètre dans la sonde jusqu'à la partie supérieure de la bougie en céramique poreuse.

En particulier, le bouchon d'étanchéité est fixé sur la partie supérieure de la bougie en céramique poreuse, et comporte trois trous assurant le passage respectivement des trois tubes capillaires.

D'après l'invention, les moyens pour mesurer la charge hydraulique du sol ainsi que la concentration des solutés comportent une pompe péristaltique reliée à la sonde par l'intermédiaire du second tube capillaire, un robinet capillaire à trois voies, un puits de mesure de la concentration des solutés relié à la sonde par l'intermédiaire du troisième tube capillaire, une alimentation électrique assurant l'autonomie électrique du dispositif, un réservoir de trop-plein et un réservoir d'eau bi-distillée utilisée pour le rinçage et la purge du circuit hydraulique.

Avantageusement, les tubes capillaires sont fabriqués dans un matériau rigide et imperméable à l'air, et la canne guide est fabriquée dans un matériau opaque protégeant l'intérieur de ladite canne guide de la lumière solaire.

Selon un mode préféré de l'invention, la bougie en céramique poreuse est remplie de billes d'un matériau chimiquement inerte, assurant ainsi un meilleur temps de réponse de la mesure.

D'autres caractéristiques et avantages de l'invention ressortiront mieux de la description qui va suivre en référence aux dessins dans lesquels :
- la figure 1, déjà décrite, représente schématiquement un tensiomètre selon l'art antérieur ;
- la figure 2 représente schématiquement la sonde selon l'invention ;
- la figure 3 représente un schéma du dispositif de mesure assurant l'autonomie du dispositif selon l'invention ;
- la figure 4 représente une bougie en céramique poreuse selon un mode préféré de réalisation de l'invention.

La figure 2 représente un schéma de la sonde selon l'invention. Cette sonde 1 comporte, comme dans l'art antérieur, une canne guide 2 en PVC opaque sur laquelle est montée une bougie 4 en céramique poreuse. Un bouchon 6 d'étanchéité est fixé, par collage époxy, sur l'extrémité supérieure 10a de la bougie. Le rôle de ce bouchon 6 d'étanchéité est de préserver l'intérieur de la canne guide 2 du soluté dont la bougie est impregnée.

La caractéristique importante de la sonde 1 selon l'invention consiste en l'existence de trois tubes capillaires T, P et U en nylon. Le bouchon 6 d'étanchéité comporte trois trous permettant le passage des trois tubes capillaires T, P et U. Le premier tube capillaire T relie le manomètre 8 à l'extrémité inférieure 10b de la bougie. Ainsi relié au fond de la bougie 4 en céramique poreuse, le manomètre permet de mesurer la charge hydraulique du soluté dont la bougie 4 est imprégnée. Le second tube capillaire U plonge également jusqu'au fond de la bougie 4 ; à son extrémité supérieure, une vanne 12U permet l'ouverture ou la fermeture du tube capillaire U. De même, l'extrémité supérieure du tube capillaire P est relié à une vanne 12P permettant l'ouverture ou la fermeture du tube capillaire P ; l'extrémité inférieure dudit tube capillaire P pénètre dans la sonde 1 jusqu'à l'extrémité supérieure 10a de la bougie 4.

Au moment de la mise en fonction du dispositif, la bougie 4 en céramique poreuse et les trois tubes capillaires T, U et P sont remplis d'une eau bi-distillée désaérée, les second et troisième tubes capillaires U et P étant fermés par les vannes respectivement 12U et 12P. L'eau bi-distillée se met en équipotentiel hydraulique avec la solution du sol et les ions de la solution du sol diffusent vers l'eau du circuit hydraulique (4, T, U, P).

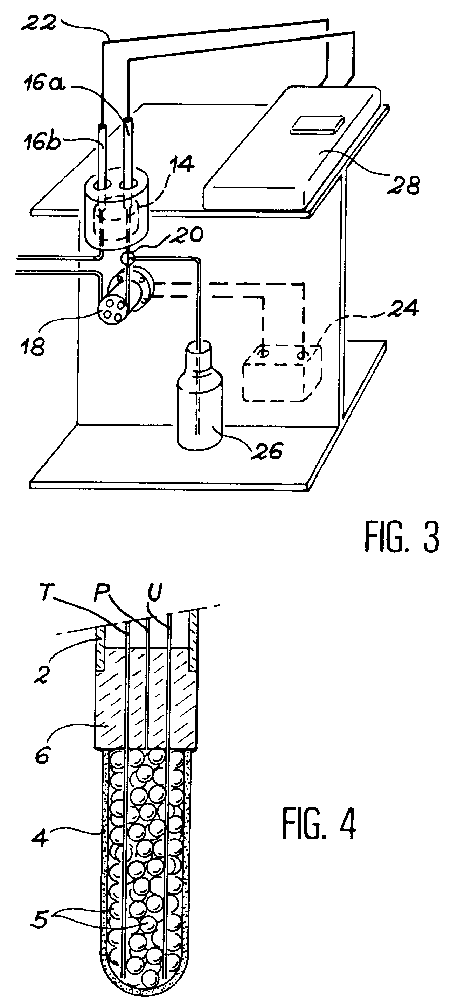

Sur la figure 3, on a représenté le dispositif de mesure de la solution contenue dans la bougie 4 en céramique poreuse. Ce dispositif de mesure comprend une pompe péristaltique 18 reliée au second tube capillaire U et permettant d'amener la solution à mesurer de la bougie 4 vers un robinet capillaire 20 à trois voies par l'intermédiaire du tube capillaire U. Le débit créé par la pompe péristaltique 18 assure une turbulence à la verticale d'une électrode 16a, les deux électrodes de mesure 16a et 16b plongeant à l'intérieur d'un puits de mesure 14 et étant reliées à un appareil d'enregistrement des mesures 28.

Si la quantité de solution amenée est trop importante, l'excédent de solution est retourné, par l'intermédiaire du trop-plein capillaire 22, vers le troisième tube capillaire P. Lorsque la mesure est terminée, le sens de rotation de la pompe péristaltique 18 est inversé à partir de l'alimentation électrique 24. La solution excédente est alors renvoyée vers la bougie 4 à travers le tube capillaire U.

Le robinet capillaire 20 permet également de prélever une partie aliquote de solution à des fins d'analyse chimique et isotopique, puis de rincer et de purger la bougie 4 et les trois tubes capillaires T, U et P avec de l'eau bi-distillée contenue dans une réserve 26.

La figure 4 représente une bougie en céramique poreuse selon un mode préféré de réalisation de l'invention. La bougie 4 en céramique poreuse est remplie de billes 5 d'un matériau chimiquement inerte, tel que le quartz, ces billes 5 permettant de diminuer le volume interne de la bougie 4. Le volume interne de la bougie 4 étant largement diminué, le temps de mise en équipotentiel hydraulique et de diffusion des ions se trouve réduit d'un facteur 2 à 4, permettant ainsi un meilleur temps de réponse de la mesure.

Outre les avantages décrits précédemment, le dispositif selon l'invention assure une autonomie électrique, permettant ainsi au dispositif d'être portable et donc utilisable sur le terrain.

## Revendications

1. Dispositif de mesure de la charge hydraulique et de la concentration ionique du sol, comprenant une sonde (1) comportant une canne guide (2), une bougie (4) en céramique poreuse montée à l'extrémité inférieure de la canne guide, un bouchon (6) d'étanchéité et un dispositif manomètrique (8), et des moyens, associés à ladite sonde, pour mesurer la charge hydraulique du sol ainsi que la concentration des solutés, caractérisé en ce que la sonde comporte au moins trois tubes capillaires (T, U et P) plongeant à l'intérieur de ladite sonde jusqu'à la bougie en céramique poreuse, un premier tube capillaire (T) servant à mesurer la charge hydraulique, un second tube capillaire (U) assurant l'extraction du soluté à étudier et un troisième tube capillaire (P) servant à purger de son air un circuit hydraulique comprenant lesdits tubes capillaires et la bougie en céramique poreuse.

2. Dispositif selon la revendication 1, caractérisé en ce que :
- le premier tube capillaire (T), relié par une extrémité supérieure au dispositif manomètrique, pénètre jusque dans la partie inférieure de la bougie en céramique poreuse ;
- le second tube capillaire (U), relié par une extrémité supérieure à une vanne (12U) assurant la fermeture ou l'ouverture dudit second tube capillaire, pénètre jusque dans la partie inférieure de la bougie en céramique poreuse ;
- le troisième tube capillaire (P), relié par une extrémité supérieure à une vanne (12P), assurant la fermeture ou l'ouverture dudit troisième tube capillaire, pénètre dans la sonde jusqu'à la partie supérieure (10a) de la bougie en céramique poreuse.

3. Dispositif selon l'une quelconque des revendications précédentes, caractérisé en ce que le bouchon d'étanchéité est fixé sur la partie supérieure de la bougie en céramique poreuse, et en ce qu'il comporte trois trous assurant le passage respectivement des trois tubes capillaires.

4. Dispositif selon l'une quelconque des revendications précédentes, caractérisé en ce que les moyens pour mesurer la charge hydraulique du sol ainsi que la concentration des solutés comportent une pompe péristaltique (18) reliée à la sonde par l'intermédiaire du second tube capillaire (U), un robinet capillaire (20) à trois voies, un puits de mesure (14) de la concentration des solutés relié à la sonde par l'intermédiaire du troisième tube capillaire (P), une alimentation électrique (24) assurant l'autonomie électrique du dispositif, un réservoir de trop-plein (22) et un réservoir (26) d'eau bi-distillée utilisée pour le rinçage et la purge du circuit hydraulique.

5. Dispositif selon l'une quelconque des revendications précédentes, caractérisé en ce que les tubes capillaires sont fabriqués dans un matériau rigide et imperméable à l'air.

6. Dispositif selon l'une quelconque des revendications précédentes, caractérisé en ce que la canne guide est fabriquée dans un matériau opaque protégeant l'intérieur de ladite canne guide de la lumière solaire.

7. Dispositif selon l'une quelconque des revendications précédentes, caractérisé en ce que la bougie en céramique poreuse est remplie de billes (5) d'un matériau chimiquement inerte, assurant ainsi un meilleur temps de réponse de la mesure.

## Patentansprüche

1. Vorrichtung zur Messung des Porenwasserdrucks und der Ionenkonzentration des Bodens, umfassend eine ein Führungsrohr (2) umfassende Sonde (1), eine Kerze (4) aus poröser Keramik, die an dem inneren Ende des Führungsrohrs angebracht ist, und ein Abdichtungsstopfen (6) und eine Manometervorrichtung (8) und mit der genannten Sonde verbundene Einrichtungen, um den Porenwasserdruck des Bodens sowie die Konzentration gelöster Stoffe zu messen, **dadurch gekennzeichnet**, daß die Sonde mindestens drei Kapillarröhren (T, U und P) umfaßt, die in das Innere der genannten Sonde bis zu der Kerze aus poröser Keramik eintauchen, wobei eine erste Kapillarröhre (T) dient, den Porenwasserdruck zu messen, eine zweite Kapillarröhre (U) die Gewinnung des zu untersuchenden gelösten Stoffes sicherstellt und eine dritte Kapillarröhre (P) dient, einen hydraulischen Kreis von seiner Luft zu entlüften, der die genannten Kapillarröhren und die Kerze aus poröser Keramik umfaßt.

2. Vorrichtung gemäß Anspruch 1, **dadurch gekennzeichnet,** daß:
- die erste Kapillarröhre (T), die durch ein oberes Ende mit der Manometervorrichtung verbunden ist, bis in den unteren Teil der Kerze aus poröser Keramik eindringt;
- die zweite Kapillarröhre (U), die mit einem oberen Ende mit einem Schieber (12 U) verbunden ist, der das Schließen oder Öffnen der genannten zweiten Kapillarröhre sicherstellt, bis in den unteren Teil der Kerze aus poröser Keramik eindringt;
- die dritte Kapillarröhre (P), die mit einem oberen Ende mit einem Schieber (12 P) verbunden ist, der das Schließen oder Öffnen der genannten dritten Kapillarröhre sicherstellt, in die Sonde bis zu dem oberen Teil (10 a) der Kerze aus poröser Keramik eindringt.

3. Vorrichtung gemäß irgendeinem der vorhergehenden Ansprüche **dadurch gekennzeichnet**, daß der Dichtungsstopfen an dem oberen Teil der Kerze aus poröser Keramik angebracht ist, und daß er drei Löcher umfaßt, die den jeweiligen Durchtritt der drei Kapillarröhren sicherstellen.

4. Vorrichtung gemäß irgendeinem der vorhergehenden Ansprüche, **dadurch gekennzeichnet**, daß die Einrichtungen zum Messen des Porenwasserdrucks sowie der Konzentration der gelösten Stoffe umfassen eine peristaltische Pumpe (18), die mit der Sonde mittels der zweiten Kapillarröhre (U) verbunden ist, einen kapillaren Dreiwegehahn (20), einen Meßschacht (14) für die Konzentration der gelösten Stoffe, der mit der Sonde über die dritte Kapillarröhre (T) verbunden ist, eine elektrische Versorgung (24), die die elektrische Unabhängigkeit der Vorrichtung sicherstellt, ein Überlaufgefäß (22) und ein Gefäß (26) für doppelt destilliertes Wasser, das zum Spülen und Reinigen des hydraulischen Kreises verwendet wird.

5. Vorrichtung gemäß irgendeinem der vorhergehenden Ansprüche, **dadurch gekennzeichnet**, daß die Kapillarröhren aus einem steifen und luftundurchlässigen Material hergestellt sind.

6. Vorrichtung gemäß irgendeinem der vorhergehenden Ansprüche, **dadurch gekennzeichnet**, daß das Führungsrohr aus einem lichtundurchlässigen Material hergestellt ist, das das Innere des genannten Führungsrohrs gegen das Sonnenlicht schützt.

7. Vorrichtung gemäß irgendeinem der vorhergehenden Ansprüche, **dadurch gekennzeichnet**, daß die Kerze aus poröser Keramik mit Kugeln (5) aus einem chemisch inerten Material gefüllt ist, die somit eine bessere Ansprechzeit bei der Messung sicherstellen.

## Claims

1. Device for measuring the pore water pressure and the ionic concentration of the soil, comprising a probe (1) including a guide pipe (2), a porous ceramic bulb (4) mounted at the lower end of the guide pipe, a sealing plug (6) and a manometric device (8), and means, associated with the said probe, for measuring the pore water pressure of the soil as well as the solute concentration, characterized in that the probe includes at least three capillary tubes (T, U and P) descending inside the said probe as far as the porous ceramic bulb, a first capillary tube (T) being used to measure the pore water pressure, a second capillary tube (U) providing extraction of the solute to be studied and a third capillary tube (P) being used to purge the air contained in a hydraulic circuit comprising the said capillary tubes and the porous ceramic bulb.

2. Device according to Claim 1, characterized in that:
- the first capillary tube (T), connected via an upper end to the manometric device, penetrates as far as the lower part of the porous ceramic bulb;
- the second capillary tube (U), connected via an upper end to a valve (12U) which closes or opens the said second capillary tube, penetrates as far as the lower part of the porous ceramic bulb;
- the third capillary tube (P), connected via an upper end to a valve (12P) which closes or opens the said third capillary tube, penetrates into the probe as far as the upper part (10a) of the porous ceramic bulb.

3. Device according to any one of the preceding claims, characterized in that the sealing plug is fixed onto the upper part of the porous ceramic bulb, and in that it includes three holes allowing passage respectively for the three capillary tubes.

4. Device according to any one of the preceding claims, characterized in that the means for measuring the pore water pressure of the soil as well as the solute concentration include a peristaltic pump (18) connected to the probe via the second capillary tube (U), a three-way capillary tap (20), a chamber (14) for measuring the solute concentration, connected to the probe via the third capillary tube (P), an electrical power supply (24) providing electrical autonomy of the device, an overfill reservoir (22) and a reservoir (26) for the double-distilled water used for rinsing and purging the hydraulic circuit.

5. Device according to any one of the preceding claims, characterized in that the capillary tubes are made of a rigid material which is impermeable to air.

6. Device according to any one of the preceding claims, characterized in that the guide pipe is made of an opaque material which protects the interior of the said guide pipe from sunlight.

7. Device according to any one of the preceding claims, characterized in that the porous ceramic bulb is filled with balls (5) made of a chemically inert material, thus ensuring a better measurement response time.
